# EUROPEAN PATENT APPLICATION

(11) **EP 1 088 887 A1**
(43) Date of publication of application: **04.04.2001**
(21) Application number: 99203117.9
(22) Date of filing: 23.09.1999
(51) Int. Cl.: C12N 9/96, C12N 9/84

(54) **Crosslinked enzyme aggregates**

(71) Applicant: DSM N.V., 6411 TE Heerlen (NL)
(72) Inventor: Cao, Linqiu, 2624 PP Delft (NL); Langen, Lukas Michael, 2613 SL Delft (NL); Janssen, Michiel Hubertus Arnold, 2611 BH Delft (NL); Sheldon, Roger Arthur, 2281 VA Rijswijk (NL)

(57) **Abstract**

Crosslinked enzyme aggregate obtainable with the process comprising the steps of (i) treating an enzyme solution, preferably an enzyme solution of a penicillin acylase, with the aid of a precipitating agent whereby aggregates are formed and precipitated, and (ii) subjecting the precipitate in situ to crosslinking with the aid of a crosslinking agent. Preferably the crosslinked enzyme aggregate has a stability factor of at least 1.5 times as high as the stability factor of the original enzyme solution, at 60°C.

Preferably, the precipitating agent is preferably chosen from ammonium sulphate, poly(ethyleneglycol) or tert-butylalcohol, and glutaric aldehyde as the crosslinking agent.

## Description

The invention relates to crosslinked enzyme aggregates with high activity, obtainable by a process comprising the steps of (i) treating an enzyme solution with the aid of a precipitating agent whereby aggregates are formed and precipitated and (ii) subjecting the precipitate in situ to crosslinking with the aid of a crosslinking agent.

It has turned out that the crosslinked enzyme aggregates according to the invention have high activity. Moreover the enzyme solution may be less purified in terms of protein content. The purity of the enzyme solution, therefore, is a matter of choice. However, when considering the aim of manufacturing a high active enzyme preparation, it is common knowledge that a more pure initial enzyme preparation will result in a more active enzyme preparation in terms of units per g of enzyme preparation in the end.

Crosslinked enzymes are described in US-A-4665028. However the actual crosslinked enzymes disclosed therein are prepared from dried a enzyme preparation that is added to a salt solution, in order to prevent dissolution of the enzyme preparations and to prevent the enzyme preparations from undesired aggregation during the crosslinking reaction. This process requires an extra drying step. In general this leads to a substantial decrease of activity.

The crosslinked enzyme aggregates according to the invention are for instance very well suited to be employed in the enzymatic acylation of β-lactamnuclei to β-lactam antibiotics, for example cephalexin, ampicillin, cefaclor, amoxicillin, cephradine, cefadroxil, cefotaxime, cefazolin, cefprozil, loracarbef and cefaloglycin. In such processes enzymes known as penicillin amidases or penicillin acylases as described for instance in WO-A-99/20786 are used.

It has appeared that the crosslinked enzyme aggregates here surprisingly have an improved thermal stability.

In particular the invention relates to crosslinked enzyme aggregates with improved thermal stability relative to the original enzyme solution from which the crosslinked enzyme aggregates are prepared, expressed in terms of stability factor (i.e. the half-life time value t_{1/2} of the crosslinked enzyme aggregates e.g. at 60°C relative to the t_{1/2} value of the original enzyme solution, of at least 1.5, preferably at least 2.0.

The invention also relates to a process for the preparation of crosslinked enzyme aggregates from an enzyme solution which process comprises the steps of (i) treating an enzyme solution with the aid of a precipitating agent whereby aggregates are formed and precipitated, and (ii) subjecting the precipitate in situ to crosslinking with the aid of a crosslinking agent.

Suitable enzymes/enzyme preparations are for instance Penicillin acylases, lipases, esterases, epoxidehydrolases, amidases, aminopeptidases, nitrilases, nitrilhydratases, glycosidases, hydantoinases or carbamoylases.

The process of the present invention consists basically of two reactions that can be easily performed in 1 pot. First, the enzyme in solution is precipitated using a precipitating agent to form insoluble enzyme aggregates. Second, the insoluble enzyme aggregates are submitted to crosslinking by a crosslinking agent to fix the structure of the insoluble enzyme aggregates. The term "enzyme aggregate" as such refers in the framework of this invention to any associated enzyme (or protein) particle obtained by any physical technique.

A preferred embodiment of the preparation of a cross-linked enzyme aggregate (CLEA) according to the invention is as follows. Precipitation is preferably performed at a temperature between 0 and 4°C for reasons of stability but can also be performed at room temperature, and is performed in a suitable buffer with a pH suitable for the enzyme of interest. In practice the pH often will be between 4-11, preferably between 5-9. The precipitating agent is usually stepwise added and/or dissolved with constant stirring and pH control when needed. The mixture is subsequantly allowed to stand for some time, for instance 15 min to 1 hour. The crosslinking agent is gently added to the mixture and again left for some time (30 min to 12 hours). When the crosslinking reaction is finished, more water may be added to lower the viscosity of the mixture and the insoluble CLEA particles can be filtered or separated by any other method and extensively washed before put in the final buffered solution for storage.

Suitable precipitating agents that can be used in the procedure of the present invention are in principle all water soluble precipitating agents that are used in the art of precipitation of biomolecules, for instance salts (organic or inorganic), organic solvents, (bio)polymers. It goes without saying that it is recommendable not to use precipitating agents that have a negative effect on enzyme stability. Some generally applicable and well known precipitating agents are quarternary ammonium salts or one of the other alkali metals, from the group of phosphate, sulfate, citrate, bicarbonate, carbonate, acetate, tartrate, succinate, chloride, and nitrate, or organic solvents like methanol, ethanol, propanol, isopropanol, butanol, and t-butylalcohol, or acetone, or any of the poly(ethylene glycol) (PEG) series. In principle, also polyamides or polyamines can be used, for instance protamine sulphate, although they may be less preferred as they may tend to react with glutaraldehyde during the crosslinking process, which might be undesirable. Preferably ammonium sulphate or a poly(ethyleenglycol) (PEG) with a molecular weight preferably between 3000 and 16000 is used.

The amount of precipitating agent to be used is calculated with respect to the final volume and expressed as % of the saturation concentration when using initially solid precipitating agents like salts or a PEG series, whereas organic solvents are expressed as % v/v. The optimal amount of precipitating agent to be used is largely dependend on the specific precipitating agent chosen, and can easily be determined by the skilled person. For instance, the preferred amount of ammonium sulphate is 10 to 80% of the saturation concentration; the preferred amount of PEG is 20-50% (w/v) and of t-butylalcohol is 50-70% (v/v). Preferably the precipitating agent is used in a concentration up to its saturation concentration.

Suitable crosslinking agents to be used are in principle all agents that can be used in the crosslinking of enzymes. The preferred crosslinking agent is glutaraldehyde. It was shown that for penicillin acylase best results were obtained when a glutaraldehyde concentration of 0.5-4% (w/v) was used. However, the preferred glutaraldehyde concentration is 0.5 - 1% (w/v) calculated with respect to the final volume.

If desired, the aggregation may be performed in the presence of a carrier. In principle all insoluble carriers are suitable since only a surface, whether internal or external, is needed. Suitable carriers are inorganic or organic synthetic or natural carriers consisting of the following components such as acrylic polymers, activated carbon, agar, agarose, alginate, celite, cellulose, chitin, chitosan, DEAE-cellulose, gelatin, glass, hydroxyapatite, kieselguhr, latex beads, magnetic particles, polyacrylamide, polypropylene, polystyrene, polyurethane, poly(vinyl alcohol), poly(vinyl chloride), polyvinylpolypyrolidone, silica, Teflon, and derivatives of all these.

The invention will be further elucidated by means of the following examples, without however being restricted thereto.

| Abbreviations: | |
|---|---|
| 7-ADCA | 7-aminodesacetoxycephalosporanic acid |
| 6-APA | 6-amino-penicillanic acid |
| AMPI | ampicillin |
| CEX | cephalexin |
| PG | D-phenylglycine |
| PGA | D-phenylglycine amide |
| GAH | Glutaric aldehyde |

### Experimental Methods

### Hydrolytic activity

The enzyme activity of the samples was measured by addition of the sample of definite volume or weight to 25 ml 100 mM phosphate buffer, pH 8.0 containing 2% (v/v) penicillin acylase. During the assay the solution was thermostated at 25°C. The solution was automatically titrated with 0.1 NaOH to the desired pH (8.0) before the same was added to the solution using a Metrohm Dosimat, a Metrohm impulsomat 614 and a Methrohm pH-meter 654 (Titrino, Metrohm, Switzerland). The liberated phenylacetic acid was neutralised by the 0.1 N NaOH. The penicillin acylase activity is then expressed in units (U). One unit corresponds to the liberation of 1 µmol phenylacetic acid per minute.

### HPLC-Analysis

All synthetic reaction mixtures were analysed by a HPLC system equipped with a Waters M6000A pump, a Nucleosil C18 10 µm 8x100mm column, a Spectra Physics SP 4400 integrator and a Shimadzu SPD-6A UV-detector using an mobile phase containing 70% water, 30% acetonitrile, SDS 0.68g/L, 5 mM phosphate buffer, pH 3.0. Analysis was performed at a flow rate of 0.7 ml/minute at room temperature with a UV-detector (215nm).

### Materials

Assemblase® is immobilized penicillin acylase from E. coli ATCC 11105 as described in WO-A-97/04086. Immobilization is carried out as described in EP-A-222462, using gelatin and chitosan as gellating agents and glutaraldehyde as crosslinking agent. Sulfuric acid (95-98%) was obtained from Baker.

### Example I

### CLEAs prepared with ammonium sulfate

5 ml penicillin acylase solution (1008 U/ml) was diluted by addition of 5 ml 0.2 M MOPS (pH 7.0) buffer. Then 5.5 g solid ammonium sulfate was slowly added under gently stirring. After that 10 ml 55% w/v ammonium solution in 0.1 M MOPS (pH 7.0) buffer was subsequently added under gently stirring. Then solution was stirred at room temperature (RT) during 2.5 hours, then 0.4 ml GAH (25%) was added and stirred at 0°C for 4 hours. Then 30 ml distilled water was added, under stirring. The CLEA was collected by filtration; 0.95 gram wet CLEAs was obtained and then dispersed in 25 ml TRIS buffer, pH 7.5 and stored at °C for use.

The specific activity of CLEA was 2235 U/gram, the retention of the activity was 41.1%.

### Example II

### CLEAs prepared with PEG 8000

5 ml penicillin acylase solution was diluted by addition of 5 ml 0.2 M MOPS (pH 7.0) buffer. Then 4 g solid poly(ethyleenglycol) with MW 8000 (PEG 8000) was slowly added under gently stirring. After that, 10 ml 40% w/v PEG 8000 in 0.1 M MOPS (pH 7.0) buffer was consequently added under gently stirring. Then solution was stirred at RT 2.5 hours, then 0.4 ml GAH (25%) was added and stirred at 0°C for 4 hours. Then 30 ml distilled water was added. Under stirring. The CLEA was collected by filtration. 1.55 wet CLEAs was obtained and then dispersed in 25 ml TRIS buffer, pH 7.5 and stored at °C for use.

The specific activity of the CLEA was 2135 U/gram, the retention of the activity was 61.2%.

### Example III

### CLEAs prepared with t-butylalcohol

5 ml penicillin acylase solution was diluted by addition of 5 ml 0.2 M MOPS (pH 7.0) buffer. Then 10 ml t-butylalcohol was slowly added under gently stirring at 0°C. After that, 5 ml 60% v/v in 0.1 M MOPS (pH 7.0) buffer was subsequently added under gently stirring. Then solution was stirred at room temperature during 2.5 hours, then 0.4 GAH (25%) was added and stirred at 0°C for 4 hours. Then 30 ml distilled water was added under stirring. The CLEA was collected by filtration. 1.2 gram wet CLEAs was obtained and then dispersed in 25 ml Trid buffer, pH 7.5 and stored at °C for use.

The specific activity of the CLEA was 2986 U/gram, the retention of the activity was 55.3%.

### Preparation of Escherichia coli penicillin G acylase CLEA's

### Example IV

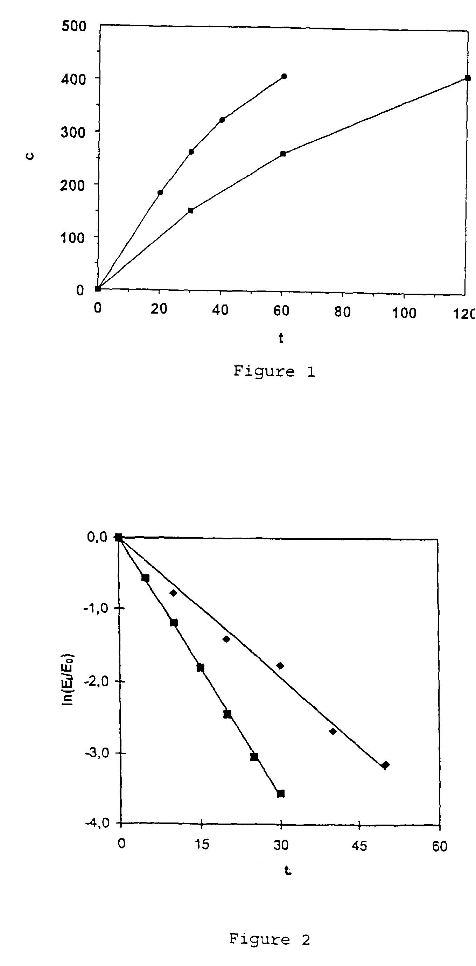

In this example the activity of CLEAs prepared according to example III, compared to Assemblase is demonstrated.
Immobilised penicillin acylase preparations (2 g) was added to a reaction mixture containing 6.8 g 7-ADCA, 4.9 g D-(-)-phenylglycine amide and 38 g water. The temperature of the reaction mixture was 3°C and the pH was adjusted to 7.6 using an ammonia solution (25 wt%). The reaction mixture was sampled and analysed by means of HPLC in order to quantify the concentration of cephalexin. The same experiment was repeated using 2.5 g Assemblase®. The results are shown in fig 1, wherein C represents the concentration of CEX (mM) and t the time (min); the symbol ● represents the results obtained with CLEA and ■ with Assemblase®.

### Comparative Experiment A

Preparation of the crosslinked enzyme according to US-A-4665028 and showing a lower activity in the same context as in Example I. The enzyme solution (145 ml, 1008 U/ml, 146160 U) was spray-dried with a 35 % activity recovery using a Büchi 190 Mini Spray Dryer (Inlet temperature 64°C (few seconds); outlet temperature 38°C (few seconds)). The spray dried enzyme was collected in a beaker. The resulting enzyme powder (1.31 g, 38.6 kU/g, 50566 U) was used as a starting material for the production of crosslinked enzyme. At 0 °C (ice), 0.1 g of the enzyme powder was added to 7 ml of a saturated Na₂SO₄ solution in phosphate buffer pH 8.0 and stirred vigorously to suspend the insoluble enzyme powder. Glutaraldehyde was added up to a final concentration of 1% (v/v) and the insoluble crosslinked enzyme particles thus formed were filtered and washed extensively with demineralized water. These crosslinked enzyme particles (0.2645 g ww, 857 U/g ww) retained 5.9 % of their original activity. The total overall recovery was 2 %.

| Spray-dried enzyme | |
|---|---|
| Amount | 1.31 g |
| Activity | 38.6 kU/g |
| Total Units | 50,566 |
| Recovery | 35 % |

| Cross-linking | |
|---|---|
| Amount dried enzyme | 0.265 g wetweight (from 0.1 g spray-powder) |
| Activity | 857 U/g wetweight |
| Total Units | 227 |
| Recovery | 5.9 % |

### Example V

This example shows the improved thermostability of CLEA particles prepared as described in Example I compared to the original *Escherichia coli* ATCC 11105 penicillin acylase enzyme solution. Both enzyme preparations were incubated at 60°C in 5 ml 50 mM phosphate buffer pH 7.0. Samples were withdrawn at 5 minute and 10 minute intervals and immediately cooled on ice. The residual enzyme activity of each sample was determined by hydrolisis of a 2% (w/v) solution of penicillin-G according to "hydrolytic activity" in the Experimental Methods part. The results are shown in Figure 2 where In (E₁/E₀) is plotted as function of the incubation time t (in min.) for the native enzyme (■) and for CLEA (◆). Both enzyme preparations obeyed first-order inactivation kinetics. The inactivation rate constant Kₐ was calculated from the slope of the linearly fitted lines of Figure 2 by plotting the natural logarithm of the quotient of the residual enzyme activity (Eₜ) and initial enzyme activity (Eₒ) against time. The stability factor of the CLEA enzyme preparation was calculated from the quotient of the half-life time values t_{1/2} of both enzyme preparations (t_{1/2} = In(2)/Kₐ, where Kₐ is the first-order inactivation rate constant calculated from the slope of the linearly fitted lines of Figure 2) and was found to be 2.

### Comparative experiment B

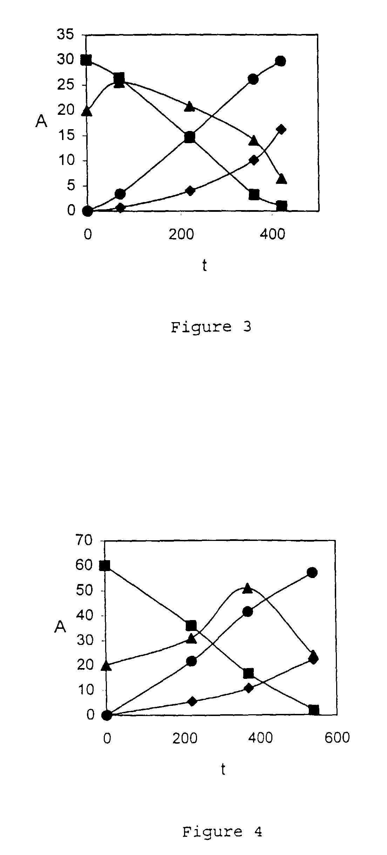

6β-aminopenicillanic acid (6.5 gram, 30 mmol)and D-phenylglycine amide (3 gram, 20 mmol) were suspended in 40 gram water at 10°C in a thermostated reaction vessel, equipped with a turbine stirrer, a pH electrode, a temperature sensor and a dropping funnel, connected to a titration device. Immobilized E. coli penicilin acylase, Assemblase® (3 gram), was added at t=0. During the reaction the pH was kept constant at 7.0, using 3M sulfuric acid. At t=60 minutes an extra portion of 1,5 gram (10 mmol) D-phenylglycine amide was added to the reaction mixture; this procedure was repeated at t=120 and t=180 minutes. During the reaction samples were taken from the reaction mixture for HPLC analysis. After 420 minutes the reaction was stopped at 97% conversion of 6-APA into ampicillin. The results are given in fig. 3 where the amount A (in mM)of PG (◆), 6-APA (■), PGA (▲) and AMPI (●) are given as a function of time (t) in min.

### Example IX

### Synthesis of Ampicillin

6β-aminopenicillanic acid (13 gram; 60 mmol) and D-phenylglycine amide (3 gram, 20 mmol) were suspended in 40 gram water at 10°C in a thermostated reaction vessel, equipped with a turbine stirrer, a pH electrode, a temperature sensor and a dropping funnel, connected to a titration device. Immobilized E. coli penicillin acylase, CLEA (1.28 gram), was added at t=0. During the raction the pH was kept constant at 7.0, using 3M sulfuric acid. At t=60 minutes an extra portion of 3 gram (20 mmol) D-phenylglycine amide was added to the reaction mixture; this procedure was repeated at t=120, t=180 and t=240 minutes. During the reaction samples were taken from reaction mixture for HPLC analysis. After 540 minutes the reaction was stopped at 95% conversion of 6-APA into ampicillin. The results are given in fig. 4 where the amount A (in min.) of PG (◆), 6-APA (■), PGA (▲) and AMPI (●) are given as a function of time (t) in min.

## Claims

1. Crosslinked enzyme aggregate obtainable with the process comprising the steps of (i) treating an enzyme solution with the aid of a precipitating agent whereby aggregates are formed and precipitated, and (ii) subjecting the precipitate in situ to crosslinking with the aid of a crosslinking agent.

2. Crosslinked enzyme aggregate according to claim 1 wherein an enzyme solution of a penicillin acylase is used.

3. Crosslinked enzyme aggregates according to claim 1 or 2 having a stability factor of at least 1.5 times as high as the stability factor of the original enzyme solution, at 60°C.

4. Process for the preparation of a crosslinked enzyme aggregate which process comprises the steps of (i) treating an enzyme solution with the aid of a precipitating agent whereby aggregates are formed and precipitated, and (ii) subjecting the precipitate in situ to crosslinking with the aid of a crosslinking agent.

5. Process according to claim 4 in which process the precipitating agent is chosen from ammonium sulphate, poly(ethyleneglycol) or tert-butylalcohol.

6. Process according of claims 4 or 5, in which process glutaric aldehyde is used as the crosslinking agent.
